# EUROPEAN PATENT APPLICATION

(11) **EP 4 825 954 A1**
(43) Date of publication of application: **21.10.2026**
(21) Application number: 24903632.8
(22) Date of filing: 06.12.2024
(51) Int. Cl.: C12M 1/00, C12M 1/06, C12N 1/00, C12N 5/00

(54) **LEARNED MODEL, ESTIMATION DEVICE, AND ESTIMATION METHOD**

(30) Priority: 12.12.2023 JP 2023209569
(71) Applicant: Chiyoda Corporation, Kanagawa 220-8765 (JP)
(72) Inventor: IRIKURA, Motoki, Yokohama-shi Kanagawa 220-8765 (JP); ISHIGAMI, Takahiro, Yokohama-shi Kanagawa 220-8765 (JP); TABATA, Kazuyoshi, Yokohama-shi Kanagawa 220-8765 (JP)
(74) Representative: Gulde & Partner
(86) International application number: PCT/JP2024/043221
(87) International publication number: WO 2025/126978

(57) **Abstract**

A trained model, an estimation device, and an estimation method capable of accurately evaluating index data related to dynamics of contents of a container are provided. The trained model is trained using at least one piece of data regarding a shape of an agitator provided in the container, data regarding a shape of a sparger provided in the container, data regarding a shape of the container, and data regarding physical properties, composition, chemical properties, biochemical properties, or concentration of contents housed in the container as an explanatory variable and using a volumetric mass transfer coefficient (kLa), an agitation power per unit volume (P/V), a respiration rate, bacterial and cell concentrations, a concentration of products derived from bacteria and cells, or a titer as a target variable.

## Description

### Technical Field

The present invention relates to a trained model, an estimation device, and an estimation method.

### Background Art

Devices called agitation tanks or bubble columns are used as reactors that dissolve components in a gas (for example, oxygen or the like) into a liquid by blowing (aerating) the gas into the liquid in a container or by vibrating the container and cause a chemical reaction with components contained in the liquid, or are used as bioreactors that culture microorganisms, animal cells, or the like in the liquid by supplying oxygen or the like or degassing carbon dioxide or the like, and are applied in petrochemical/chemical, pharmaceutical/life science fields, or the like.

In such an agitation tank or a bubble column accompanied by gas aeration, gas aerated into the liquid is dispersed in the liquid as bubbles or gas slugs, but since dissolution of gas components from the gas into the liquid or transfer of dissolved components from the liquid to the gas (mass transfer) occurs at an interface between the gas and the liquid (gas-liquid interface) in these bubbles and the gas slugs, a mass transfer rate thereof depends on a total area of the gas-liquid interfaces or a dispersion state of the bubbles and the gas slugs in the liquid. In order to improve the mass transfer rate as much as possible, device design is performed so as to increase a gas-liquid interface area and to evenly disperse the bubbles and the gas slugs in the liquid.

As this index, a volumetric mass transfer coefficient kL*a (hereinafter, kLa), which is obtained by multiplying a gas-liquid interface area concentration "a" (a gas-liquid interface area per unit liquid volume) by "kL" which is an index of a diffusion rate of gas components into the liquid, is well known (for example, see Patent Literature 1). In particular, when scaling up from a small lab scale to a large commercial scale, design verification is conducted so that kLa in a commercial device becomes equivalent to that in the lab scale. Similarly, an agitation power per unit liquid volume (P/V), a respiration rate, bacterial and cell concentrations, a concentration of products derived from bacteria and cells, a titer, and the like are also indices taken into consideration so that they are equivalent to those at the lab scale when scaled up to the commercial scale.

### Citation List

### Patent Literature

[Patent Literature 1] Japanese Unexamined Patent Application Publication No. 2001-75947

### Summary of Invention

### Technical Problem

By the way, kLa and P/V of the agitation tank or the bubble column are evaluated by correlation formulas established based on experimental results and physical considerations. However, for example, since kLa in the agitation tank is greatly affected by operating conditions of the agitator, fluid properties, and an agitation shape, there has been a problem that it is difficult to accurately evaluate kLa under all conditions using the correlation formulas. Furthermore, regarding the respiration rate, the bacterial and cell concentrations, the concentration of products derived from bacteria and cells, the titer, and the like, there has been a problem that correlation formulas for evaluating them based on the shape of the agitator or the sparger, the shape of the container, the physical properties of the contents of the tank, and the like, without relying on operation of actual equipment, have not been established, and it is difficult to accurately evaluate them.

An object of the present invention is to provide a trained model, an estimation device, and an estimation method capable of accurately evaluating index data related to dynamics of contents of the container, such as the above-mentioned kLa, P/V, respiration rate, bacterial and cell concentrations, concentration of products derived from bacteria and cells, and titer.

### Solution to Problem

According to the present invention, a trained model having the following configuration is provided.
[1] A trained model, wherein the trained model is trained using at least one piece of data regarding a shape of an agitator provided in a container, data regarding a shape of a sparger provided in the container, data regarding a shape of the container, and data regarding physical properties, composition, chemical properties, biochemical properties, or concentration of contents housed in the container as an explanatory variable, and using a volumetric mass transfer coefficient (kLa), an agitation power per unit volume (P/V), a respiration rate, bacterial and cell concentrations, a concentration of products derived from bacteria and cells, or a titer as a target variable.

According to the present invention, at least one of the data regarding the shape of the agitator, the data regarding the shape of the sparger, the data regarding the shape of the container, and the data regarding the physical properties, the composition, the chemical properties, or the biochemical properties of the contents (explanatory variables) is input to the trained model, and the volumetric mass transfer coefficient (kLa), the agitation power per unit volume (P/V), the respiration rate, the bacterial and cell concentrations, the concentration of products derived from bacteria and cells, or the titer output as the target variables can be acquired; therefore, compared with the prior art, index data related to dynamics of the contents of the container can be accurately evaluated.

Hereinafter, various embodiments of the present invention will be exemplified. The embodiments shown below can be combined with each other.
[2] The trained model according to [1], wherein the trained model is trained using the data regarding the shape of the agitator, the data regarding the shape of the sparger, the data regarding the shape of the container, and the data regarding physical properties, composition, chemical properties, biochemical properties, or concentration of the contents housed in the container as explanatory variables.
[3] The trained model according to [1] or [2], wherein the data regarding the shape of the agitator includes data regarding a shape of an agitating blade constituting the agitator, the data regarding the shape of the agitating blade and the data regarding the shape of the sparger include (i) data to which a numerical label is assigned for each type of shape, or (ii) data digitized by assigning a first numerical value to regions occupied by the agitating blade and the sparger in a three-dimensional space and assigning a second numerical value to a region not occupied by the agitating blade and the sparger in the three-dimensional space.
[4] An estimation device comprising an input unit and a target variable acquisition unit, wherein the input unit inputs the explanatory variable to the trained model according to any one of [1] to [3], and the target variable acquisition unit acquires the target variable output from the trained model when the explanatory variable is input to the trained model.
[5] The estimation device according to [4], further comprising an explanatory variable acquisition unit, wherein the explanatory variable acquisition unit acquires a value of the explanatory variable input to the trained model when a value of the target variable output from the trained model equals a predetermined value.
[6] The estimation device according to [5], wherein of first data and second data constituting the explanatory variable, a value of the first data is a fixed value and a value of the second data is a variable value, and the explanatory variable acquisition unit acquires the value of the second data input to the trained model when the value of the target variable output from the trained model equals the predetermined value.
[7] An estimation method, comprising inputting the explanatory variable to the trained model according to any one of [1] to [3], and acquiring the target variable output from the trained model when the explanatory variable is input to the trained model.

According to the present invention, it is possible to accurately evaluate index data related to dynamics of contents of a container.

### Brief Description of Drawings

FIG. 1 is a block diagram showing a functional configuration of an estimation device according to an embodiment of the present invention.
FIG. 2 is a flowchart showing an example of estimation processing according to the embodiment of the present invention.
FIG. 3 is an image diagram showing changes in values of output data according to cultivation days.

### Description of Embodiments

Embodiments of the present invention will be described with reference to the accompanying drawings. In addition, in each of the drawings, components given the same reference signs have the same or similar configurations.

### (Configuration of Estimation Device 10)

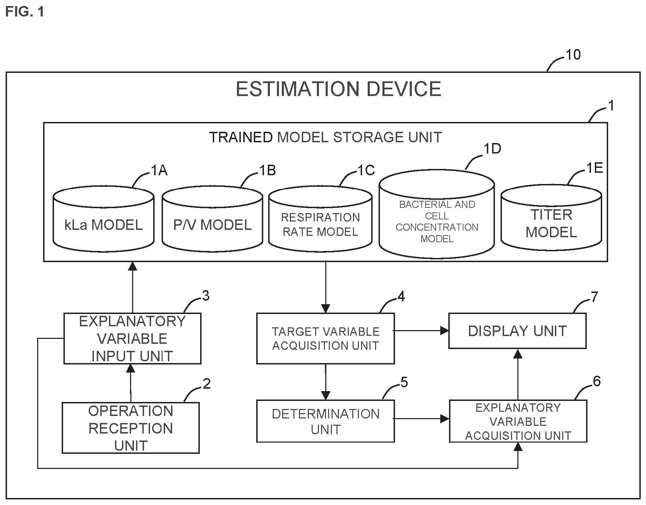

FIG. 1 is a block diagram showing the functional configuration of the estimation device 10 in the present embodiment. The estimation device 10 estimates (acquires) kLa (a volumetric mass transfer coefficient), P/V (an agitation power per unit liquid volume), a respiration rate, bacterial and cell concentrations, a concentration of products derived from bacteria and cells, or a titer as index data related to dynamics of contents of the container by using a trained model. Here, mass transfer refers to dissolution of components in a gas (for example, oxygen or the like) into a liquid or transfer of dissolved components from the liquid to the gas by blowing (aerating) the gas into the liquid in a container or by vibrating the container in an agitation tank or a bubble column accompanied by gas aeration.

The agitation tank and the bubble column are used as a reactor that dissolves components in a gas into a liquid and causes a chemical reaction with components contained in the liquid, or as a bioreactor that cultures microorganisms, animal cells, or the like in the liquid by supplying oxygen or the like or degassing carbon dioxide or the like, and are applied in petrochemical/chemical, pharmaceutical/life science fields, or the like.

As shown in FIG. 1, the estimation device 10 comprises a trained model storage unit 1, an operation reception unit 2, an explanatory variable input unit 3, a target variable acquisition unit 4, a determination unit 5, an explanatory variable acquisition unit 6, and a display unit 7. The explanatory variable input unit 3 functions as an "input unit" of the present invention.

Each component of the estimation device 10 may be realized by software or may be realized by hardware. In a case where the components are realized by software, various functions can be realized by a CPU executing a computer program. The program may be stored in a built-in storage unit or may be stored in a computer-readable non-transitory recording medium. Alternatively, a program stored in an external storage unit may be read to realize the components by so-called cloud computing. In a case where the components are realized by hardware, they can be realized by various circuits such as an ASIC, an FPGA, or a DRP (Dynamically Reconfigurable Processor). In the present embodiment, various pieces of information or concepts including them are handled, and these are represented by high or low signal values as a binary bit set composed of 0s or 1s, and communication or calculation can be executed by aspects of the software or the hardware described above.

The trained model storage unit 1 is a database that stores a kLa model 1A, a P/V model 1B, a respiration rate model 1C, a bacterial and cell concentration model 1D, and a titer model 1E as trained models machine-trained by a machine learning unit (not shown).

The machine learning unit performs machine learning by using learning data (training data) stored in a learning data storage unit (not shown). That is, the machine learning unit generates the trained model by inputting multiple sets of learning data to a learning model to cause the learning model to machine-learn (so-called supervised learning) a correlation between input data and output data constituting the learning data. For example, a CNN (Convolutional Neural Network) or the like is used as the trained model. As a learning algorithm for machine learning, for example, deep learning or the like is used.

Note that, although the present embodiment describes the trained model as being a CNN, the trained model is not limited to a CNN and may be a model trained by an arbitrary learning algorithm such as a neural network other than a CNN, an SVM (Support Vector Machine), a Bayesian network, or a regression tree.

The kLa model 1A is a trained model trained using, for example, data regarding a shape of an agitator provided in a container, data regarding a shape of a sparger provided in the container, data regarding a shape of the container, and data regarding physical properties, composition, chemical properties, biochemical properties, or concentration of contents housed in the container as input data (explanatory variables) constituting the learning data, and using kLa (a volumetric mass transfer coefficient) as output data (a target variable) constituting the learning data.

Data regarding the shape of the agitator is, for example, data regarding a shape of an agitating blade constituting the agitator, such as a type of the agitating blade, a diameter of the agitating blade, or a number of the agitating blades set according to the shape of the agitating blade (paddles (pitched/flat), disk turbines (pitched/flat), propellers, anchors, or the like). The data regarding the shape of the agitating blade includes data to which a numerical label is assigned for each type of shape, or includes data digitized by assigning a first numerical value (for example, 1) to regions occupied by the agitating blade in a three-dimensional space and assigning a second numerical value (for example, 0) to a region not occupied by the agitating blade in the three-dimensional space. The shape data of the agitating blade digitized by the first numerical value and the second numerical value is represented as a single numerical value by, for example, taking an average value and a standard deviation across an entire device, and can be used as an explanatory variable in a learning model. Moreover, a method of assigning a numerical label for each type of the shape of the agitating blade and a method of numerical representation by assigning the first numerical value (for example, 1) to the regions occupied by the agitating blade in the three-dimensional space and assigning the second numerical value (for example, 0) to the region not occupied by the agitating blade in the three-dimensional space may be combined. In this case, by assigning a numerical label for each type of the shape of the agitating blade (the paddle (pitched/flat), the disk turbine (pitched/flat), the propeller, the anchor, or the like) and further representing presence or absence of occupation of the agitating blade in the three-dimensional space with numerical values, the shape of the agitating blade can be more precisely represented as numerical data.

Data regarding the shape of the sparger provided in the container includes, for example, a type of an aeration sparger, an aeration sparger diameter, an aeration sparger hole diameter, or a number of aeration sparger holes set according to the shape of the sparger (a ring shape, a cylindrical shape, a circular shape). The data regarding the shape of the sparger includes data to which a numerical label is assigned for each type of shape, or includes data digitized by assigning a first numerical value (for example, 1) to a region occupied by the sparger in a three-dimensional space and assigning a second numerical value (for example, 0) to a region not occupied by the sparger in the three-dimensional space.

Data regarding the shape of the container is data regarding a shape of the container itself that houses the contents. The data regarding the shape of the container includes, for example, a type of the shape of the container (a cylindrical type, a rectangular type, or the like), dimensions of the container (an inner diameter (in the case of a cylinder), a width (in the case of a rectangle), a height, or the like), presence or absence of a baffle (in the case of the agitation tank), or the like.

Data regarding physical properties, composition, chemical properties, biochemical properties, or concentration regarding the contents includes, for example, material-specific physical properties, composition, chemical properties, and biochemical properties included in the contents and physical properties of the entire contents in the container and a concentration of each material contained in the contents.

The material-specific physical properties included in the contents include, for example, a material-specific density, viscosity, elasticity, non-Newtonian properties, or the like. The material-specific composition included in the contents includes, for example, elements constituting the material and content thereof, ions dissolved in the material and content thereof, presence or absence of a surfactant, a content of sugars, a content of amino acids, a content of vitamins, a content of inorganic salts, a content of lipid components, a content of trace metals, or the like. The material-specific chemical properties included in the contents include, for example, reaction activity, activation energy, a reaction rate, a reaction rate constant, or the like in a chemical reaction of the material. The material-specific biochemical properties included in the contents include, for example, presence or absence of mycelium formation or a doubling time (in the case where the material is a cell line), or a growth factor contained (in the case where the material is a culture medium). The materials included in the contents are, for example, a culture medium and cells, and in that case, as the material-specific physical properties included in the contents, fluid physical properties of the culture medium (a density, viscosity, a non-Newtonian properties, or the like), a medium composition (presence or absence of a surfactant, a type of growth factor, a concentration of sugars contained in the medium, a concentration of amino acids contained in the medium, a concentration of vitamins contained in the medium, a concentration of inorganic salts contained in the medium, a concentration of lipid components contained in the medium, a concentration of trace metals contained in the medium, or the like), or a type of a cell line/strain (a mass, viscosity, elasticity, a size, a shape, presence or absence of mycelium formation, a doubling time, or the like) are used as explanatory variables of the learning model. The material-specific physical properties, composition, chemical properties, and biochemical properties included in the contents are parameters that do not change even after the device starts operating including the contents, or parameters whose values taken under operating conditions such as temperature or pressure are known.

The physical properties of the entire contents in the container include, for example, a temperature, osmotic pressure, an oxidation-reduction potential (ORP), or pH of the contents. The concentration of each material contained in the contents includes, for example, a seeding amount, a cell concentration, or the like (in the case where the contents are a culture medium and cells). The data regarding the physical properties of the entire contents in the container used as the explanatory variable of the learning model may be an initial value before starting operation of the container, may be a predicted value obtained by simulating the state after starting the operation of the container (for example, a numerical fluid dynamics simulation, such as a CFD (Computational Fluid Dynamics) simulation), or may be a measured value continuously acquired after starting the operation of the container. For example, the cell concentration or the pH is an initial value set by a value input by a user, a predicted value by the CFD (Computational Fluid Dynamics) simulation (an in-container average value plus a standard deviation value), or set by an average value within the device plus a standard deviation value obtained from a multipoint measurement method after starting the operation.

As learning data of the trained model, from among the data regarding the physical properties, the composition, the chemical properties, the biochemical properties, or the concentration of the contents housed in the container, it is preferable to particularly include the material-specific physical properties, composition, chemical properties, and biochemical properties included in the contents and initial values of the physical properties of the entire contents in the container and the concentration of each material contained in the contents. Since this data can be obtained without operation of actual equipment, they can be easily acquired as the explanatory variables for the trained model in order to predict performance of the actual equipment in device design. More preferably, in addition to the material-specific physical properties, composition, chemical properties, and biochemical properties included in the contents and the initial values of the physical properties of the entire contents in the container and the concentration of each material contained in the contents, it is preferable to further include values predicted by simulation for the physical properties of the entire contents in the container and the concentration of each material contained in the contents. By including not only the initial values of these values but also values after the device operation in the explanatory variables, values of target variables can be more accurately estimated.

In another example, in addition to the material-specific physical properties, composition, chemical properties, and biochemical properties included in the contents, the initial values of the physical properties of the entire contents in the container and the concentration of each material contained in the contents, and the values predicted by simulation for the physical properties of the entire contents in the container and the concentration of each material contained in the contents, it is preferable to further include measured values of the physical properties of the entire contents in the container and the concentration of each material contained in the contents acquired during operation of the actual equipment. Since the physical properties of the entire contents in the container and the concentration of each material contained in the contents are values that vary continuously according to operation of the device, by inputting these pieces of data as the explanatory variables during the operation, the trained model can be used for evaluation of the actual equipment operation.

The kLa model 1A may be trained using a superficial gas velocity, an aeration rate, a turbulent energy dissipation rate, a gas hold-up, a bubble diameter, a control method for dissolved oxygen (DO) (an upper limit value, a lower limit value, or the like), a rotation speed of an agitator, culture medium feeding/harvesting conditions, shear stress, and a feed amount/perfusion amount of the culture medium (in the case of fed-batch culture/perfusion culture) as input data (explanatory variables) constituting the learning data, and using kLa (a volumetric mass transfer coefficient) as output data (a target variable) constituting the learning data. By configuring the kLa model 1A to include one or more of these parameters in the input data, prediction accuracy of kLa can be further improved.

The superficial gas velocity is a set value calculated from an aeration rate and a tank diameter input by a user. The aeration rate, the rotation speed, the control method for dissolved oxygen, and the feed amount/perfusion amount (the culture medium feeding/harvesting conditions) are set by values input by the user. The turbulent energy dissipation rate is a value theoretically calculated from P/V (agitation power per unit volume) or an average value within the device plus a standard deviation value obtained from a CFD simulation or a multipoint measurement method in an experiment. The gas hold-up is a value calculated from a liquid level difference between aerated and non-aerated conditions, or an average value within the device plus a standard deviation value obtained from a CFD simulation or a multipoint measurement method in an experiment. The bubble diameter is a value measured from a captured image or the like, or an average value within the device plus a standard deviation value obtained from a CFD simulation or a multipoint measurement method in an experiment. The shear stress is set by a value input by the user or set by an average value within the device plus a standard deviation value obtained from a CFD simulation or a multipoint measurement method in an experiment. Moreover, the explanatory variables of the kLa model 1A may include kLa approximately estimated by a conventional approximate expression for approximately deriving kLa. By including this in the explanatory variables, in the kLa model 1A, evaluation can be performed by also taking into account a correlation between kLa estimated by the approximate expression and kLa output by the model 1A.

Learning data of kLa, which is the target variable of the kLa model 1A, is acquired by a publicly known method. kLa may be obtained experimentally in actual equipment (for example, a static method of measuring a change in a dissolved oxygen concentration in liquid with an oxygen electrode provided in a container and analyzing an increase curve of the dissolved oxygen concentration), or may be obtained by a CFD simulation. Preferably, the learning data is configured with data experimentally obtained in the actual equipment, but in the case where the data from the actual equipment is insufficient, the learning data preferably includes data obtained by the CFD simulation in addition to the data experimentally obtained in the actual equipment. Note that, since the trained model according to the present invention is a model that substitutes for a conventional approximate expression for obtaining kLa, kLa obtained by a conventional correlation formula without experiment using the actual equipment is preferably not included in the learning data of the target variable of the kLa model 1A.

The P/V model 1B is a trained model trained using, for example, data regarding a shape of an agitator provided in a container and data regarding physical properties, composition, chemical properties, biochemical properties, or concentration of contents housed in the container as input data (explanatory variables) constituting the learning data, and using P/V (an agitation power per unit liquid volume) as output data (a target variable) constituting the learning data.

Data regarding the shape of the agitator is, for example, data regarding a shape of an agitating blade constituting the agitator, such as a power number or a diameter of the agitating blade set according to the shape of the agitating blade. The data regarding the shape of the agitating blade includes data to which a numerical label is assigned for each type of shape, or includes data digitized by assigning a first numerical value (for example, 1) to a region occupied by the agitating blade in a three-dimensional space and assigning a second numerical value (for example, 0) to a region not occupied by the agitating blade in the three-dimensional space. Data regarding the physical properties, the composition, the chemical properties, or the biochemical properties of the contents includes, for example, a culture medium density set according to the culture medium.

The P/V model 1B may be trained using data regarding a shape of a sparger provided in a container, data regarding a shape of the container, an operating parameter of an agitator, or aeration conditions as input data (explanatory variables) constituting the learning data, and using P/V as output data (a target variable) constituting the learning data. Here, as the operating parameter of the agitator, for example, a rotation speed set by a value input by a user is exemplified. Moreover, as the aeration conditions, for example, an aeration flow rate set by a value input by the user is exemplified. Furthermore, the explanatory variables of the P/V model 1B may include P/V approximately estimated by a conventional approximate expression for approximately deriving P/V (such as Michel-Miller empirical formula). By including this in the explanatory variables, in the P/V model 1B, evaluation can be performed by also taking into account a correlation between P/V estimated by the approximate expression and P/V output by the model 1B.

Learning data of P/V, which is the target variable of the P/V model 1B, is acquired by a publicly known method. For example, it is obtained by measuring electric power required for operation of an agitator as agitation power in actual equipment and dividing the power by the volume of the container. Similarly to the kLa model 1A, the P/V model 1B also preferably does not include P/V obtained by a conventional correlation formula using the actual equipment in the learning data of the target variable.

The respiration rate model 1C is a trained model trained using, for example, data regarding a shape of an agitator provided in a container, data regarding a shape of a sparger provided in the container, data regarding a shape of the container, and data regarding physical properties, composition, chemical properties, biochemical properties, or concentration of contents housed in the container as input data (explanatory variables) constituting the learning data, and using a respiration rate as output data (a target variable) constituting the learning data.

Data regarding the shape of the agitator is, for example, data regarding a shape of an agitating blade constituting the agitator, such as a type of the agitating blade, a diameter of the agitating blade, or a number of the agitating blades set according to the shape of the agitating blade. The data regarding the shape of the agitating blade includes data to which a numerical label is assigned for each type of shape, or includes data digitized by assigning a first numerical value (for example, 1) to a region occupied by the agitating blade in a three-dimensional space and assigning a second numerical value (for example, 0) to a region not occupied by the agitating blade in the three-dimensional space.

Data regarding the shape of the sparger provided in the container includes, for example, a type of an aeration sparger, an aeration sparger diameter, an aeration sparger hole diameter, or a number of aeration sparger holes set according to the shape of the sparger. The data regarding the shape of the sparger includes data to which a numerical label is assigned for each type of shape, or includes data digitized by assigning a first numerical value (for example, 1) to a region occupied by the sparger in a three-dimensional space and assigning a second numerical value (for example, 0) to a region not occupied by the sparger in the three-dimensional space.

Data regarding the shape of the container is data regarding a shape of the container itself that houses the contents. The data regarding the shape of the container includes, for example, a type of the shape of the container (a cylindrical type, a rectangular type, or the like), dimensions of the container (an inner diameter (in the case of a cylinder), a width (in the case of a rectangle), a height, or the like), presence or absence of a baffle (in the case of the agitation tank), or the like.

Data regarding physical properties, composition, chemical properties, biochemical properties, or concentration regarding the contents includes, for example, material-specific physical properties, composition, chemical properties, and biochemical properties included in the contents and physical properties of the entire contents in the container and a concentration of each material contained in the contents.

The respiration rate model 1C may be trained using a superficial gas velocity, an aeration rate, a turbulent energy dissipation rate, a gas hold-up, a bubble diameter, a control method for dissolved oxygen (DO) (an upper limit value, a lower limit value, or the like), a rotation speed of an agitator, shear stress, and a feed amount/perfusion amount of the culture medium (in the case of fed-batch culture/perfusion culture) as input data (explanatory variables) constituting the learning data, and using a respiration rate as output data (a target variable) constituting the learning data. By configuring the respiration rate model 1C to include one or more of these parameters in the input data, prediction accuracy of the respiration rate can be further improved.

The superficial gas velocity is a set value calculated from an aeration rate and a tank diameter input by a user. The aeration rate, the rotation speed, the control method for dissolved oxygen, and the feed amount/perfusion amount (the culture medium feeding/harvesting conditions) are set by values input by the user. The turbulent energy dissipation rate is a value theoretically calculated from P/V (agitation power per unit volume) or an average value within the device plus a standard deviation value obtained from a CFD simulation or a multipoint measurement method in an experiment. The gas hold-up is a value calculated from a liquid level difference between aerated and non-aerated conditions, or an average value within the device plus a standard deviation value obtained from a CFD simulation or a multipoint measurement method in an experiment. The bubble diameter is a value measured from a captured image or the like, or an average value within the device plus a standard deviation value obtained from a CFD simulation or a multipoint measurement method in an experiment. The shear stress is set by a value input by the user or set by an average value within the device plus a standard deviation value obtained from a CFD simulation or a multipoint measurement method in an experiment.

Learning data of a respiration rate, which is the target variable of the respiration rate model 1C, is acquired by a publicly known method. For example, the learning data of the respiration rate is calculated from an oxygen concentration and an exhaust gas concentration measured in actual equipment during operation.

The bacterial and cell concentration model 1D is a trained model trained using, for example, data regarding a shape of an agitator provided in a container, data regarding a shape of a sparger provided in the container, data regarding a shape of the container, and data regarding physical properties, composition, chemical properties, biochemical properties, or concentration of contents housed in the container as input data (explanatory variables) constituting the learning data, and using bacterial and cell concentrations as output data (a target variable) constituting the learning data.

Data regarding the shape of the agitator is, for example, data regarding a shape of an agitating blade constituting the agitator, such as a type of the agitating blade, a diameter of the agitating blade, or a number of the agitating blades set according to the shape of the agitating blade. The data regarding the shape of the agitating blade includes data to which a numerical label is assigned for each type of shape, or includes data digitized by assigning a first numerical value (for example, 1) to a region occupied by the agitating blade in a three-dimensional space and assigning a second numerical value (for example, 0) to a region not occupied by the agitating blade in the three-dimensional space.

Data regarding the shape of the sparger provided in the container includes, for example, a type of an aeration sparger, an aeration sparger diameter, an aeration sparger hole diameter, or a number of aeration sparger holes set according to the shape of the sparger. The data regarding the shape of the sparger includes data to which a numerical label is assigned for each type of shape, or includes data digitized by assigning a first numerical value (for example, 1) to a region occupied by the sparger in a three-dimensional space and assigning a second numerical value (for example, 0) to a region not occupied by the sparger in the three-dimensional space.

Data regarding the shape of the container is data regarding a shape of the container itself that houses the contents. The data regarding the shape of the container includes, for example, a type of the shape of the container (a cylindrical type, a rectangular type, or the like), dimensions of the container (an inner diameter (in the case of a cylinder), a width (in the case of a rectangle), a height, or the like), presence or absence of a baffle (in the case of the agitation tank), or the like.

Data regarding physical properties, composition, chemical properties, biochemical properties, or concentration regarding the contents includes, for example, material-specific physical properties, composition, chemical properties, and biochemical properties included in the contents and physical properties of the entire contents in the container and a concentration of each material contained in the contents.

The bacterial and cell concentration model 1D may be trained using a superficial gas velocity, an aeration rate, a turbulent energy dissipation rate, a gas hold-up, a bubble diameter, a control method for dissolved oxygen (DO) (an upper limit value, a lower limit value, or the like), a rotation speed of an agitator, shear stress, a feed amount/perfusion amount of the culture medium (in the case of fed-batch culture/perfusion culture), addition timing of a fed-batch medium (in the case of fed-batch culture/perfusion culture), a fed-batch culture method, cultivation days, a cell (bacterial) survival rate, and a viable cell (bacterial) density as input data (explanatory variables) constituting the learning data, and using bacterial and cell concentrations as output data (a target variable) constituting the learning data.

The superficial gas velocity is a set value calculated from an aeration rate and a tank diameter input by a user. The aeration rate, the rotation speed, the control method for dissolved oxygen, and the feed amount/perfusion amount are set by values input by the user. The turbulent energy dissipation rate is a value theoretically calculated from P/V (agitation power per unit volume) or an average value within the device plus a standard deviation value obtained from a CFD simulation or a multipoint measurement method in an experiment. The gas hold-up is a value calculated from a liquid level difference between aerated and non-aerated conditions, or an average value within the device plus a standard deviation value obtained from a CFD simulation or a multipoint measurement method in an experiment. The bubble diameter is a value measured from a captured image or the like, or an average value within the device plus a standard deviation value obtained from a CFD simulation or a multipoint measurement method in an experiment. The shear stress is set by a value input by the user or set by an average value within the device plus a standard deviation value obtained from a CFD simulation or a multipoint measurement method in an experiment.

Further, the bacterial and cell concentration model 1D may use a concentration of products derived from bacteria and cells as a target variable in addition to or instead of the bacterial and cell concentrations. The products from bacteria and cells include proteins, genes, or the like produced by the bacteria and cells and secreted from the bacteria and cells. The proteins include a target production substance (an antibody or the like) and an inhibitor that inhibits growth or the like of cells.

Learning data of bacterial and cell concentrations and/or a concentration of products derived from bacteria and cells, which are target variables of the bacterial and cell concentration model 1D, are acquired by a publicly known method. For example, these learning data include bacterial and cell concentrations and/or a concentration of products derived from bacteria and cells measured by sampling a part of contents in actual equipment during operation or measured by an optical sensor.

The titer model 1E is a trained model trained using, for example, data regarding a shape of an agitator provided in a container, data regarding a shape of a sparger provided in the container, data regarding a shape of the container, and data regarding physical properties, composition, chemical properties, biochemical properties, or concentration of contents housed in the container as input data (explanatory variables) constituting the learning data, and using a titer as output data (a target variable) constituting the learning data.

Data regarding the shape of the agitator is, for example, data regarding a shape of an agitating blade constituting the agitator, such as a type of the agitating blade, a diameter of the agitating blade, or a number of the agitating blades set according to the shape of the agitating blade. The data regarding the shape of the agitating blade includes data to which a numerical label is assigned for each type of shape, or includes data digitized by assigning a first numerical value (for example, 1) to a region occupied by the agitating blade in a three-dimensional space and assigning a second numerical value (for example, 0) to a region not occupied by the agitating blade in the three-dimensional space.

Data regarding the shape of the sparger provided in the container includes, for example, a type of an aeration sparger, an aeration sparger diameter, an aeration sparger hole diameter, or a number of aeration sparger holes set according to the shape of the sparger. The data regarding the shape of the sparger includes data to which a numerical label is assigned for each type of shape, or includes data digitized by assigning a first numerical value (for example, 1) to a region occupied by the sparger in a three-dimensional space and assigning a second numerical value (for example, 0) to a region not occupied by the sparger in the three-dimensional space.

Data regarding the shape of the container is data regarding a shape of the container itself that houses the contents. The data regarding the shape of the container includes, for example, a type of the shape of the container (a cylindrical type, a rectangular type, or the like), dimensions of the container (an inner diameter (in the case of a cylinder), a width (in the case of a rectangle), a height, or the like), presence or absence of a baffle (in the case of the agitation tank), or the like.

Data regarding physical properties, composition, chemical properties, biochemical properties, or concentration regarding the contents includes, for example, material-specific physical properties, composition, chemical properties, and biochemical properties included in the contents and physical properties of the entire contents in the container and a concentration of each material contained in the contents.

The titer model 1E may be trained using a superficial gas velocity, an aeration rate, a turbulent energy dissipation rate, a gas hold-up, a bubble diameter, a control method for dissolved oxygen (DO) (an upper limit value, a lower limit value, or the like), a rotation speed of an agitator, a feed amount/perfusion amount of the culture medium (in the case of fed-batch culture/perfusion culture), shear stress, addition timing of a fed-batch medium (in the case of fed-batch culture/perfusion culture), a fed-batch culture method, cultivation days, a cell (bacterial) survival rate, and a viable cell (bacterial) density as input data (explanatory variables) constituting the learning data, and using a titer as output data (a target variable) constituting the learning data.

The superficial gas velocity is a set value calculated from an aeration rate and a tank diameter input by a user. The aeration rate, the rotation speed, the control method for dissolved oxygen, and the feed amount/perfusion amount are set by values input by the user. The turbulent energy dissipation rate is a value theoretically calculated from P/V (agitation power per unit volume) or an average value within the device plus a standard deviation value obtained from a CFD simulation or a multipoint measurement method in an experiment. The gas hold-up is a value calculated from a liquid level difference between aerated and non-aerated conditions, or an average value within the device plus a standard deviation value obtained from a CFD simulation or a multipoint measurement method in an experiment. The bubble diameter is a value measured from a captured image or the like, or an average value within the device plus a standard deviation value obtained from a CFD simulation or a multipoint measurement method in an experiment. The shear stress is set by a value input by the user or set by an average value within the device plus a standard deviation value obtained from a CFD simulation or a multipoint measurement method in an experiment.

Learning data of a titer, which is the target variable of the titer model 1E, is acquired by a publicly known method. For example, the learning data of the titer includes a value of a titer measured by sampling a part of contents in actual equipment during operation or measured by an optical sensor.

The operation reception unit 2 is configured by, for example, a keyboard, a mouse, or the like, and receives input of various operations by a user. In the present embodiment, the operation reception unit 2 selects a kLa model 1A, a P/V model 1B, a respiration rate model 1C, bacterial and cell concentration model 1D, or a titer model 1E stored in the trained model storage unit 1, and receives a setting operation from the user for setting input data (explanatory variables) to be input to the selected model. Then, the operation reception unit 2 outputs setting operation information indicating content of the received setting operation (a model, input data) to the explanatory variable input unit 3. Here, the operation reception unit 2 may receive the input data one set at a time, or may receive a plurality of data sets at the same time. In a case where a plurality of data sets are received at the same time, for example, the operation reception unit 2 is configured to receive sets of numerical values for each input data piece by receiving range settings for each data set.

In a case where a model indicated by the setting operation information output from the operation reception unit 2 is the kLa model 1A, the explanatory variable input unit 3 inputs the input data indicated by the setting operation information to the kLa model 1A. Here, the input data input to the kLa model 1A is input data constituting learning data used for learning of the kLa model 1A, that is, data regarding a shape of an agitator provided in a container, data regarding a shape of a sparger provided in the container, data regarding a shape of the container, and data regarding physical properties, composition, chemical properties, biochemical properties, or concentration of contents housed in the container.

In a case where a model indicated by the setting operation information output from the operation reception unit 2 is the P/V model 1B, the explanatory variable input unit 3 inputs the input data indicated by the setting operation information to the P/V model 1B. Here, the input data input to the P/V model 1B is input data constituting learning data used for learning of the P/V model 1B, that is, data regarding a shape of an agitator provided in a container, data regarding a shape of the container, and data regarding physical properties, composition, chemical properties, biochemical properties, or concentration of contents housed in the container.

In a case where a model indicated by the setting operation information output from the operation reception unit 2 is the respiration rate model 1C, the explanatory variable input unit 3 inputs the input data indicated by the setting operation information to the respiration rate model 1C. Here, the input data input to the respiration rate model 1C is input data constituting learning data used for learning of the respiration rate model 1C, that is, data regarding a shape of an agitator provided in a container, data regarding a shape of the container, data regarding a shape of a sparger provided in the container, and data regarding physical properties, composition, chemical properties, biochemical properties, or concentration of contents housed in the container.

In a case where a model indicated by the setting operation information output from the operation reception unit 2 is the bacterial and cell concentration model 1D, the explanatory variable input unit 3 inputs the input data indicated by the setting operation information to the bacterial and cell concentration model 1D. Here, the input data input to the bacterial and cell concentration model 1D is input data constituting learning data used for learning of the bacterial and cell concentration model 1D, that is, data regarding a shape of an agitator provided in a container, data regarding a shape of a sparger provided in the container, data regarding a shape of the container, and data regarding physical properties, composition, chemical properties, biochemical properties, or concentration of contents housed in the container.

In a case where a model indicated by the setting operation information output from the operation reception unit 2 is the titer model 1E, the explanatory variable input unit 3 inputs the input data indicated by the setting operation information to the titer model 1E. Here, the input data input to the titer model 1E is input data constituting learning data used for learning of the titer model 1E, that is, data regarding a shape of an agitator provided in a container, data regarding a shape of a sparger provided in the container, data regarding a shape of the container, and data regarding physical properties, composition, chemical properties, biochemical properties, or concentration of contents housed in the container.

When the input data is input to the kLa model 1A by the explanatory variable input unit 3, the target variable acquisition unit 4 acquires kLa output from the kLa model 1A. Then, the target variable acquisition unit 4 outputs information indicating the acquired kLa to the display unit 7 to display the kLa. Accordingly, a user viewing the display unit 7 can accurately evaluate kLa, which is one of the pieces of index data related to dynamics of the contents of the container, compared with the prior art in which evaluation is performed by using a correlation formula. Moreover, the target variable acquisition unit 4 outputs information indicating the acquired kLa to the determination unit 5.

When the input data is input to the P/V model 1B by the explanatory variable input unit 3, the target variable acquisition unit 4 acquires P/V output from the P/V model 1B. Then, the target variable acquisition unit 4 outputs information indicating the acquired P/V to the display unit 7 to display the P/V. Accordingly, the user viewing the display unit 7 can accurately evaluate P/V, which is one of the pieces of index data related to dynamics of the contents of the container, compared with the prior art in which evaluation is performed by using a correlation formula. Moreover, the target variable acquisition unit 4 outputs information indicating the acquired P/V to the determination unit 5.

When the input data is input to the respiration rate model 1C by the explanatory variable input unit 3, the target variable acquisition unit 4 acquires a respiration rate output from the respiration rate model 1C. Then, the target variable acquisition unit 4 outputs information indicating the acquired respiration rate to the display unit 7 to display the respiration rate. Accordingly, the user viewing the display unit 7 can accurately evaluate a respiration rate, which is one of the pieces of index data related to dynamics of the contents of the container, which was conventionally difficult to evaluate without operation of actual equipment. Moreover, the target variable acquisition unit 4 outputs information indicating the acquired respiration rate to the determination unit 5.

When the input data is input to the bacterial and cell concentration model 1D by the explanatory variable input unit 3, the target variable acquisition unit 4 acquires bacterial and cell concentrations output from the bacterial and cell concentration model 1D. Then, the target variable acquisition unit 4 outputs information indicating the acquired bacterial and cell concentrations to the display unit 7 to display the bacterial and cell concentrations. Accordingly, the user viewing the display unit 7 can accurately evaluate bacterial and cell concentrations, which is one of the pieces of index data related to dynamics of the contents of the container, which was conventionally difficult to evaluate without operation of actual equipment. Moreover, the target variable acquisition unit 4 outputs information indicating the acquired bacterial and cell concentrations to the determination unit 5.

When the input data is input to the titer model 1E by the explanatory variable input unit 3, the target variable acquisition unit 4 acquires a titer output from the titer model 1E. Then, the target variable acquisition unit 4 outputs information indicating the acquired titer to the display unit 7 to display the titer. Accordingly, the user viewing the display unit 7 can accurately evaluate a titer, which is one of the pieces of index data related to dynamics of the contents of the container, which was conventionally difficult to evaluate without operation of actual equipment. Moreover, the target variable acquisition unit 4 outputs information indicating the acquired titer to the determination unit 5.

The determination unit 5 determines whether or not a value of kLa output from the target variable acquisition unit 4 equals a predetermined value and outputs information indicating a determination result to the explanatory variable acquisition unit 6. Here, the predetermined value is, for example, a desired value of kLa designed so that kLa in a commercial device becomes equivalent to that in a lab scale when scaling up from a small lab scale to a large commercial scale, and may be a single value or may be a plurality of values.

The determination unit 5 determines whether or not a value of P/V output from the target variable acquisition unit 4 equals a predetermined value and outputs information indicating a determination result to the explanatory variable acquisition unit 6. Here, the predetermined value is, for example, a desired value of P/V designed so that P/V in a commercial device becomes equivalent to that in a lab scale when scaling up from a small lab scale to a large commercial scale, and may be a single value or may be a plurality of values.

The determination unit 5 determines whether or not a value of respiration rate output from the target variable acquisition unit 4 equals a predetermined value and outputs information indicating a determination result to the explanatory variable acquisition unit 6. Here, the predetermined value is, for example, a desired value of respiration rate designed so that respiration rate in a commercial device becomes equivalent to that in a lab scale when scaling up from a small lab scale to a large commercial scale, and may be a single value or may be a plurality of values.

The determination unit 5 determines whether or not a value of bacterial and cell concentrations output from the target variable acquisition unit 4 equals a predetermined value and outputs information indicating a determination result to the explanatory variable acquisition unit 6. Here, the predetermined value is, for example, a desired value of bacterial and cell concentrations designed so that bacterial and cell concentrations in a commercial device become equivalent to those at a lab scale when scaling up from a small lab scale to a large commercial scale, and may be a single value or may be a plurality of values.

The determination unit 5 determines whether or not a value of a titer output from the target variable acquisition unit 4 equals a predetermined value and outputs information indicating a determination result to the explanatory variable acquisition unit 6. Here, the predetermined value is, for example, a desired value of a titer designed so that the titer in a commercial device becomes equivalent to that at a lab scale when scaling up from a small lab scale to a large commercial scale, and may be a single value or may be a plurality of values.

The explanatory variable acquisition unit 6 refers to a determination result output from the determination unit 5, and in the case where the value of kLa output from the target variable acquisition unit 4 equals a predetermined value, the explanatory variable acquisition unit 6 acquires input data (explanatory variables) input to the kLa model 1A when the value of kLa was output from the kLa model 1A and outputs information indicating the acquired input data to the display unit 7 to display the input data. Accordingly, the user viewing the display unit 7 can easily grasp the input data without searching for the input data with which the value of kLa equals the desired value by trial and error.

The explanatory variable acquisition unit 6 refers to a determination result output from the determination unit 5, and in the case where the value of P/V output from the target variable acquisition unit 4 equals a predetermined value, the explanatory variable acquisition unit 6 acquires input data (explanatory variables) input to the P/V model 1B when the value of P/V was output from the P/V model 1B and outputs information indicating the acquired input data to the display unit 7 to display the input data. Accordingly, the user viewing the display unit 7 can easily grasp the input data without searching for the input data with which the value of P/V equals the desired value by trial and error.

The explanatory variable acquisition unit 6 refers to a determination result output from the determination unit 5, and in the case where the value of respiration rate output from the target variable acquisition unit 4 equals a predetermined value, the explanatory variable acquisition unit 6 acquires input data (explanatory variables) input to the respiration rate model 1C when the value of respiration rate was output from the respiration rate model 1C and outputs information indicating the acquired input data to the display unit 7 to display the input data. Accordingly, the user viewing the display unit 7 can easily grasp the input data without searching for the input data with which the value of respiration rate equals the desired value by trial and error.

The explanatory variable acquisition unit 6 refers to a determination result output from the determination unit 5, and in the case where the value of bacterial and cell concentrations output from the target variable acquisition unit 4 equals a predetermined value, the explanatory variable acquisition unit 6 acquires input data (explanatory variables) input to the bacterial and cell concentration model 1D when the value of bacterial and cell concentrations was output from the bacterial and cell concentration model 1D and outputs information indicating the acquired input data to the display unit 7 to display the input data. Accordingly, the user viewing the display unit 7 can easily grasp the input data without searching for the input data with which the value of bacterial and cell concentrations equals the desired value by trial and error.

The explanatory variable acquisition unit 6 refers to a determination result output from the determination unit 5, and in the case where the value of a titer output from the target variable acquisition unit 4 equals a predetermined value, the explanatory variable acquisition unit 6 acquires input data (explanatory variables) input to the titer model 1E when the value of a titer was output from the titer model 1E and outputs information indicating the acquired input data to the display unit 7 to display the input data. Accordingly, the user viewing the display unit 7 can easily grasp the input data without searching for the input data with which the value of a titer equals the desired value by trial and error.

The display unit 7 is configured by, for example, a touch panel display provided in the estimation device 10 and displays various pieces of information output from the target variable acquisition unit 4 and the explanatory variable acquisition unit 6.

### (Estimation Processing Performed by Estimation Device 10)

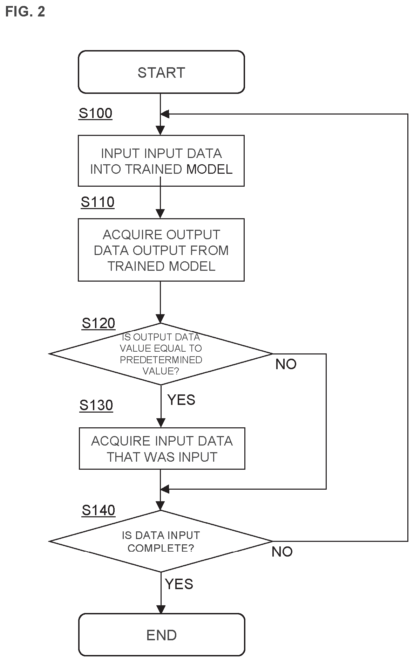

FIG. 2 is a flowchart showing an example of estimation processing performed by the estimation device 10 in the present embodiment. Processing of step S100 in FIG. 2 starts when the operation reception unit 2 selects a kLa model 1A, a P/V model 1B, respiration rate model 1C, bacterial and cell concentration model 1D, or a titer model 1E stored in the trained model storage unit 1 and receives a setting operation from a user for setting input data (explanatory variables) to be input to the selected model.

First, the explanatory variable input unit 3 inputs input data set via the operation reception unit 2 to the trained model (the kLa model 1A, the P/V model 1B, the respiration rate model 1C, the bacterial and cell concentration model 1D, or the titer model 1E) selected via the operation reception unit 2 (step S100).

Next, the target variable acquisition unit 4 acquires output data (target variables: kLa, P/V, respiration rate, bacterial and cell concentrations, a concentration of products derived from bacteria and cells, or a titer) output from the trained model when the input data is input to the trained model by the explanatory variable input unit 3 and causes the display unit 7 to display the acquired output data (step S110).

Next, the determination unit 5 determines whether or not a value of the output data acquired by the target variable acquisition unit 4 equals a predetermined value (step S120). As a result of the determination, in the case where the value of the output data does not reach the predetermined value (step S120, NO), processing transitions to step S140.

On the other hand, in the case where the value of the output data equals the predetermined value (step S120, YES), the explanatory variable acquisition unit 6 acquires input data (explanatory variables) input to the trained model when the value of the output data was output from the trained model and causes the display unit 7 to display the acquired input data (step S130). After that, the processing transitions to step S140.

In step S140, the explanatory variable input unit 3 determines whether or not input of the input data set via the operation reception unit 2 to the trained model (the kLa model 1A, the P/V model 1B, the respiration rate model 1C, the bacterial and cell concentration model 1D, or the titer model 1E) selected via the operation reception unit 2 has ended. As a result of the determination, in the case where the input of the input data has not ended (step S140, NO), the processing returns to before step S100. On the other hand, in the case where the input of the input data has ended (step S140, YES), the estimation device 10 ends the estimation processing shown in FIG. 2.

### (Effects of the Present Embodiment)

As described in detail above, in the present embodiment, the estimation device 10 comprises the explanatory variable input unit 3 and the target variable acquisition unit 4. The explanatory variable input unit inputs explanatory variables (input data) to the trained model (the kLa model 1A, the P/V model 1B, the respiration rate model 1C, the bacterial and cell concentration model 1D, or the titer model 1E). The target variable acquisition unit 4 acquires target variables (output data) output from the trained model when the explanatory variables are input to the trained model. The trained model is trained using at least one piece of data regarding a shape of an agitator provided in a container, data regarding a shape of a sparger provided in the container, data regarding a shape of the container, and data regarding physical properties, composition, chemical properties, biochemical properties, or concentration of contents housed in the container as explanatory variables when gas is blown into liquid in the container, and using a volumetric mass transfer coefficient (kLa), an agitation power per unit volume (P/V), a respiration rate, bacterial and cell concentrations, a concentration of products derived from bacteria and cells, or a titer as a target variable.

According to the present embodiment configured as such, at least one piece of data regarding a shape of an agitator, data regarding a shape of a sparger, data regarding a shape of a container, and data regarding physical properties, composition, chemical properties, biochemical properties, or concentration regarding contents (explanatory variables) is input to a trained model, and a volumetric mass transfer coefficient (kLa), an agitation power per unit volume (P/V), a respiration rate, bacterial and cell concentrations, a concentration of products derived from bacteria and cells, or a titer output as target variables can be acquired; therefore, compared with the prior art, index data related to dynamics of contents of the container can be accurately evaluated. For example, conventional correlation formulas for a volumetric mass transfer coefficient (kLa) and an agitation power per unit volume (P/V) consider only some parameters such as an aeration rate or a rotation speed of an agitator, and did not take into account influences regarding shapes of the agitator and a sparger and physical properties, composition, chemical properties, biochemical properties, and concentration of contents of a container. Therefore, in scale-up design of a device, even if calculations by correlation formulas showed results in which a volumetric mass transfer coefficient (kLa) and an agitation power per unit volume (P/V) satisfied predetermined values, in operation of actual equipment designed accordingly, a volumetric mass transfer coefficient (kLa) and an agitation power per unit volume (P/V) sometimes did not satisfy the predetermined values. In contrast, in the present invention, by adopting a configuration in which these parameters are calculated by a machine learning model that adds shapes of the agitator, the sparger, and the container, and physical properties, composition, chemical properties, biochemical properties, and concentration of contents of the container which exert complex influences as a device is scaled up, accurate volumetric mass transfer coefficient (kLa) and agitation power per unit volume (P/V) can be calculated.

Moreover, according to the present embodiment, by inputting at least one piece of data regarding a shape of an agitator, data regarding a shape of a sparger, data regarding a shape of a container, and data regarding physical properties, composition, chemical properties, biochemical properties, or concentration regarding contents (explanatory variables) to a trained model, respiration rate, bacterial and cell concentrations, a concentration of products derived from bacteria and cells, or a titer can be acquired as a target variable. Conventionally, it was difficult to estimate values of these parameters at a stage of designing actual equipment without relying on operation of the actual equipment. In the present invention, since these parameters can be estimated at a time of design, it becomes possible to more surely design a device that exhibits desired performance in operation of the actual equipment.

Furthermore, the trained model according to the present invention can be used not only at a time of design but also for the purpose of estimating dynamics of contents during operation of actual equipment. Regarding kLa, P/V, respiration rate, bacterial and cell concentrations, a concentration of products derived from bacteria and cells, and a titer indicating dynamics of contents, values thereof fluctuate after start of operation. In order to estimate this in real time, the trained model of the present invention can be used. In this case, parameters same as those described above may be included. In a case where the trained model according to the present invention is used for a purpose of estimating dynamics of contents during operation of the actual equipment, it is preferable to include measured values of physical properties of the entire contents in a container and a concentration of each material contained in the contents acquired during operation of the actual equipment in explanatory variables of the trained model. By continuously acquiring these during operation and inputting the values to the trained model, it becomes possible to more accurately estimate current dynamics. Moreover, captured images from a side window or an upper window of the container, measured values by an odor sensor related to contents, measured values related to vibrations of the container and accompanying sounds, a survival rate of bacteria and cells, or a density of viable bacteria and cells may be included as explanatory variables. In a case where a captured image is included as an explanatory variable, for example, information regarding a color of contents (for example, RGB values) in the captured image is extracted and used as an explanatory variable, whereby a correlation between a color of contents and indices related to dynamics of contents can be reflected in the trained model.

### (Modified Example)

Note that, in the above embodiment, of first data (for example, data regarding a shape of an agitator) and second data (for example, data regarding a shape of a sparger) constituting input data (explanatory variables) input to the trained model, a value of the first data may be a fixed value and a value of the second data may be a variable value. In this case, the explanatory variable acquisition unit 6 acquires a value of the second data input to the trained model when a value of output data (a target variable) output from the trained model equals a predetermined value. Accordingly, with a value of the first data fixed, a user can easily acquire the second data with which a value of output data output from the trained model equals a desired value without searching for the second data by trial and error. The present embodiment is useful, for example, when a constraint condition exists during design, such that an agitator to be used cannot be changed and it is necessary to design a device that satisfies a desired value of kLa by varying only a shape of a sparger.

Moreover, in the above embodiment, the operation reception unit 2 selects a plurality of models from among the kLa model 1A, the P/V model 1B, the respiration rate model 1C, the bacterial and cell concentration model 1D, and the titer model 1E stored in the trained model storage unit 1 and receives a setting operation from a user for setting input data (explanatory variables) to be input to the selected plurality of models. Here, for input data, input data to each of a plurality of models may be received respectively, or input data common to a plurality of models may be received collectively. Then, the operation reception unit 2 outputs setting operation information indicating content of the received setting operation (a model, input data) to the explanatory variable input unit 3, and the target variable acquisition unit 4 acquires outputs of each of a plurality of models. The determination unit 5 determines whether or not an output of each of a plurality of models equals a respective predetermined value. The explanatory variable acquisition unit 6 may acquire explanatory variables when all outputs of a plurality of models reach predetermined values or may acquire explanatory variables when a part of outputs of a plurality of models reach predetermined values. However, in scale-up design of a device, since it is preferable to design so that all outputs reach predetermined values, the explanatory variable acquisition unit 6 preferably acquires explanatory variables when all outputs of a plurality of models reach predetermined values. For example, in the case where a plurality of models are the kLa model 1A and the P/V model 1B, the explanatory variable acquisition unit 6 is preferably configured to acquire explanatory variables when an output of the kLa model 1A equals a predetermined value and an output of the P/V model 1B equals a predetermined value. In scale-up design of a device, since importance is attached particularly to whether or not values of kLa and P/V reach predetermined values, it is preferable that a plurality of models include at least the kLa model 1A and the P/V model 1B. More preferably, in order to ensure that a designed device achieves desired operation, a plurality of models preferably include all of the kLa model 1A, the P/V model 1B, the respiration rate model 1C, the bacterial and cell concentration model 1D, and the titer model 1E.

Moreover, in the above embodiment, a target variable of a first model selected from the kLa model 1A, the P/V model 1B, the respiration rate model 1C, the bacterial and cell concentration model 1D, and the titer model 1E may be included in explanatory variables of a second model selected from the kLa model 1A, the P/V model 1B, the respiration rate model 1C, the bacterial and cell concentration model 1D, and the titer model 1E. kLa, P/V, respiration rate, bacterial and cell concentrations, a concentration of products derived from bacteria and cells, and a titer, which are pieces of index data representing dynamics of contents, are parameters having correlations with each other. Therefore, by adopting an index estimated by one model as an explanatory variable for a model using another index as a target variable, a target variable can be more accurately estimated. For example, kLa is, as described above, an index indicating a mass transfer rate of gas into liquid, but when a mass transfer rate increases, gas uptake by bacterial cells and cells in liquid is promoted and respiration rate increases, and as a result, bacterial and cell concentrations, a concentration of products derived from bacteria and cells, and a titer can also increase. Therefore, kLa (the target variable of the first model) estimated by the kLa model 1A may be included as explanatory variables (explanatory variables of the second model) of the respiration rate model 1C, the bacterial and cell concentration model 1D, or the titer model 1E. Similarly, a value of a target variable of the P/V model 1B may be included in explanatory variables of the kLa model 1A, the respiration rate model 1C, the bacterial and cell concentration model 1D, or the titer model 1E, or a value of a target variable of the respiration rate model 1C may be included in explanatory variables of the bacterial and cell concentration model 1D or the titer model 1E.

Moreover, in the above embodiment, a bioreactor involving aeration and agitation has been described, but a target of the trained model according to the present invention is not limited to this. For example, a bioreactor involving neither aeration nor agitation (for example, a vibration bioreactor) may be a target. In this case, as explanatory variables for models 1A to 1E, data regarding a shape of an agitator and data regarding a shape of a sparger provided in a container may not be included, and data regarding a shape of the container and data regarding physical properties, composition, chemical properties, biochemical properties, or concentration of contents housed in the container may be included. Furthermore, a bioreactor not involving aeration but involving agitation may be a target, and in this case, as the explanatory variables for models 1A to 1E, data regarding a shape of a sparger provided in a container may not be included, and data regarding a shape of an agitator, data regarding a shape of the container, and data regarding physical properties, composition, chemical properties, biochemical properties, or concentration of contents housed in the container may be included. Additionally, a bioreactor involving aeration but not involving agitation may be a target, and in this case, as the explanatory variables for models 1A to 1E, data regarding a shape of an agitator may not be included, and data regarding a shape of a sparger provided in a container, data regarding a shape of the container, and data regarding physical properties, composition, chemical properties, biochemical properties, or concentration of contents housed in the container may be included. Moreover, a reactor (such as a bubble column) in which a chemical reaction (a chemical synthesis reaction, a chemical decomposition reaction, or the like) is performed inside may be a target of the trained model, and in this case as well, explanatory variables may be similarly set according to presence or absence of an agitator and presence or absence of a sparger. Note that, in this case, as data regarding physical properties, composition, chemical properties, biochemical properties, or a concentration regarding contents housed in a container, it is preferable to particularly include material-specific chemical properties included in contents (for example, reaction activity, activation energy, a reaction rate, or a reaction rate constant in a chemical reaction of a material).

In the above embodiment, output data (target variables: kLa, P/V, respiration rate, bacterial and cell concentrations, a concentration of products derived from bacteria and cells, or a titer) output from a trained model (the kLa model 1A, the P/V model 1B, the respiration rate model 1C, the bacterial and cell concentration model 1D, or the titer model 1E) may change with passage of time.

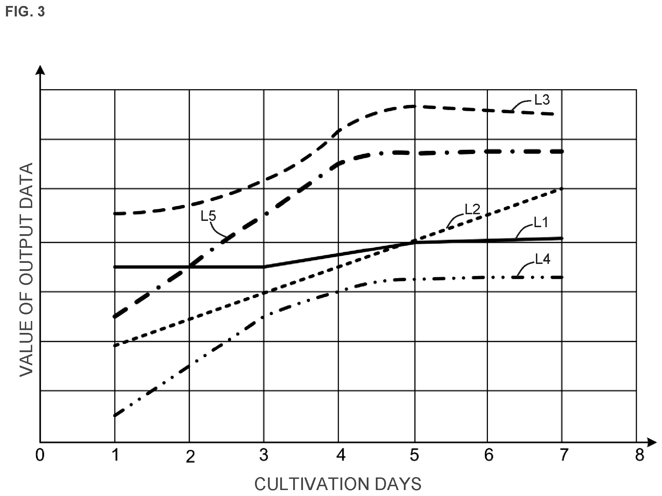

FIG. 3 is an image diagram showing changes in values of output data according to cultivation days. In FIG. 3, a curve L1 shows a change in values of output data (kLa) output from the kLa model 1A according to cultivation days (1 day to 7 days in a shown example). A curve L2 shows a change in values of output data (P/V) output from the P/V model 1B according to cultivation days. A curve L3 shows a change in values of output data (respiration rate) output from the respiration rate model 1C according to cultivation days. A curve L4 shows a change in values of output data (a cell concentration) output from the bacterial and cell concentration model 1D according to cultivation days. A curve L5 shows a change in values of output data (a titer) output from the titer model 1E according to cultivation days. As shown in FIG. 3, values of output data (P/V, particularly the cell concentration and the titer) change significantly according to the cultivation days.

Considering change in values of the output data shown in FIG. 3, values of output data (kLa, P/V, respiration rate, bacterial and cell concentrations, a concentration of products derived from bacteria and cells, or a titer) output from a trained model (the kLa model 1A, the P/V model 1B, the respiration rate model 1C, the bacterial and cell concentration model 1D, or the titer model 1E) may be a value on a final day of cultivation (7th day), may be a value on an intermediate day of cultivation (4th day), may be both the value on the final day of cultivation (7th day) and the value on the intermediate day of cultivation (4th day), or may be an average value over an entire period (7 days).

Moreover, the kLa model 1A may be trained using cultivation days in addition to data regarding a shape of an agitator, data regarding a shape of a sparger, and data regarding physical properties, composition, chemical properties, or biochemical properties regarding contents as input data (explanatory variables) constituting learning data and using kLa (a volumetric mass transfer coefficient) as output data (a target variable) constituting the learning data. In this case, the explanatory variable input unit 3 inputs input data including cultivation days to the kLa model 1A.

Moreover, the P/V model 1B may be trained using cultivation days in addition to data regarding a shape of an agitator and data regarding physical properties, composition, chemical properties, or biochemical properties regarding contents as input data (explanatory variables) constituting learning data and using P/V as output data (a target variable) constituting the learning data. In this case, the explanatory variable input unit 3 inputs input data including cultivation days to the P/V model 1B.

Moreover, the respiration rate model 1C may be trained using cultivation days in addition to data regarding a shape of an agitator, data regarding a shape of a sparger, data regarding a shape of a container, and data regarding physical properties, composition, chemical properties, or biochemical properties regarding contents as input data (explanatory variables) constituting learning data and using respiration rate as output data (a target variable) constituting the learning data. In this case, the explanatory variable input unit 3 inputs input data including cultivation days to the respiration rate model 1C.

Moreover, the bacterial and cell concentration model 1D may be trained using cultivation days in addition to data regarding a shape of an agitator, data regarding a shape of a sparger, data regarding a shape of a container, and data regarding physical properties, composition, chemical properties, or biochemical properties regarding contents as input data (explanatory variables) constituting learning data and using bacterial and cell concentrations and a concentration of products derived from bacteria and cells as output data (target variables) constituting the learning data. In this case, the explanatory variable input unit 3 inputs input data including cultivation days to the bacterial and cell concentration model 1D.

Moreover, the titer model 1E may be trained using cultivation days in addition to data regarding a shape of an agitator, data regarding a shape of a sparger, data regarding a shape of a container, and data regarding physical properties, composition, chemical properties, or biochemical properties regarding contents as input data (explanatory variables) constituting learning data and using a titer as output data (a target variable) constituting the learning data. In this case, the explanatory variable input unit 3 inputs input data including cultivation days to the titer model 1E.

Moreover, considering that values of output data (kLa, P/V, respiration rate) slightly change according to cultivation days primarily under influence of data regarding physical properties, composition, chemical properties, or biochemical properties regarding contents, it may be confirmed whether or not values of output data (kLa, P/V, respiration rate) output from a trained model (the kLa model 1A, the P/V model 1B, the respiration rate model 1C) satisfy predetermined values by inputting data regarding physical properties, composition, chemical properties, or biochemical properties regarding contents expected within the estimated cultivation days to the trained model.

Moreover, the above embodiments are merely examples of implementation in carrying out the present invention, and technical scope of the present invention should not be interpreted limitedly by these. That is, the present invention can be implemented in various forms without departing from the gist or principal features thereof.

### Reference Signs List

1: trained model storage unit, 1A: kLa model, 1B: P/V model, 1C: respiration rate model, 1D: bacterial and cell concentration model, 1E: titer model, 2: operation reception unit, 3: explanatory variable input unit, 4: target variable acquisition unit, 5: determination unit, 6: explanatory variable acquisition unit, 7: display unit, 10: estimation device

## Claims

1. A trained model, wherein
the trained model is trained using at least one piece of data regarding a shape of an agitator provided in a container, data regarding a shape of a sparger provided in the container, data regarding a shape of the container, and data regarding physical properties, composition, chemical properties, biochemical properties, or concentration of contents housed in the container as an explanatory variable, and
using a volumetric mass transfer coefficient (kLa), an agitation power per unit volume (P/V), a respiration rate, bacterial and cell concentrations, a concentration of products derived from bacteria and cells, or a titer as a target variable.

2. The trained model according to claim 1, wherein
the trained model is trained using the data regarding the shape of the agitator, the data regarding the shape of the sparger, the data regarding the shape of the container, and the data regarding physical properties, composition, chemical properties, biochemical properties, or concentration of the contents housed in the container as explanatory variables.

3. The trained model according to claim 1, wherein
the data regarding the shape of the agitator includes data regarding a shape of an agitating blade constituting the agitator,
the data regarding the shape of the agitating blade and the data regarding the shape of the sparger include (i) data to which a numerical label is assigned for each type of shape, or (ii) data digitized by assigning a first numerical value to regions occupied by the agitating blade and the sparger in a three-dimensional space and assigning a second numerical value to a region not occupied by the agitating blade and the sparger in the three-dimensional space.

4. An estimation device comprising an input unit and a target variable acquisition unit, wherein
the input unit inputs the explanatory variable to the trained model according to any one of claims 1 to 3, and
the target variable acquisition unit acquires the target variable output from the trained model when the explanatory variable is input to the trained model.

5. The estimation device according to claim 4, further comprising an explanatory variable acquisition unit, wherein
the explanatory variable acquisition unit acquires a value of the explanatory variable input to the trained model when a value of the target variable output from the trained model equals a predetermined value.

6. The estimation device according to claim 5, wherein
of first data and second data constituting the explanatory variable, a value of the first data is a fixed value and a value of the second data is a variable value, and
the explanatory variable acquisition unit acquires the value of the second data input to the trained model when the value of the target variable output from the trained model equals the predetermined value.

7. An estimation method, comprising
inputting the explanatory variable to the trained model according to any one of claims 1 to 3, and
acquiring the target variable output from the trained model when the explanatory variable is input to the trained model.
